(19) Europäisches Patentamt
European Patent Office
Office européen des brevets

(11) Publication number: **0 167 385 B1**

(12) **EUROPEAN PATENT SPECIFICATION**

(45) Date of publication of patent specification: **18.03.92**

(51) Int. Cl.$^5$: **C07D 239/46**, C07D 405/12, A61K 31/505

(21) Application number: **85304694.4**

(22) Date of filing: **01.07.85**

# (54) Antiviral compounds, treatment and formulations.

(30) Priority: **03.07.84 US 627393**
**27.10.84 GB 8427223**
**27.10.84 GB 8427224**

(43) Date of publication of application:
**08.01.86 Bulletin 86/02**

(45) Publication of the grant of the patent:
**18.03.92 Bulletin 92/12**

(84) Designated Contracting States:
**AT BE CH DE FR GB IT LI LU NL SE**

(56) References cited:
**EP-A- 0 049 072**

**CHEMICAL ABSTRACTS, vol. 99, 1983, page 678, abstract no. 212872e, Columbus, Ohio, US; K.K. OGILVIE et al.: "Synthesis of 5-substituted-1-[[2-hydroxy-1-(hydroxymethyl)ethoxy]methyl]cytosines"**

(73) Proprietor: **THE WELLCOME FOUNDATION LIMITED**
**Unicorn House 160 Euston Road**
**London NW1 2BP(GB)**

(72) Inventor: **Beauchamp, Lilia Marie**
**3007 Wycliff Road**
**Raleigh North Carolina(US)**
Inventor: **DeMiramda, Paulo Manuel Salvador**
**4828 Radcliff Road**
**Raleigh North Carolina(US)**
Inventor: **Krenitsky, Thomas Anthony**
**106 Laurel Hill Road**
**Chapel Hill North Carolina(US)**

(74) Representative: **Garrett, Michael et al**
**The Wellcome Foundation Limited Group Patents and Agreements Langley Court**
**Beckenham Kent BR3 3BS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 167 385 B1

## Description

The present invention relates to anti-viral use of certain pyrimidine derivatives and to such novel derivatives.

U.K. Patent Specification No. 1523865 and U.S. Patent No. 4,199,574 describe a broad class of purine derivatives containing an acyclic side chain in the 9-position, i.e., the position normally occupied by a sugar moiety in a purine nucleoside. These derivatives have been found to have antiviral activity against several DNA viruses of the herpes virus group, including herpes simplex, varicella zoster and Epstein Barr viruses.

Among these derivatives, 9-(2-hydroxyethoxymethyl)guanine (otherwise known as acyclovir) has been found to have particularly good activity against herpes simplex viruses both in vitro and in vivo.

On the other hand a number of pyrimidine derivatives having the same acyclic chain as acyclovir in the 1-position, i.e. the position normally occupied by a sugar moiety in a pyrimidine nucleoside, have been reported as having little or no activity against herpes simplex virus type 1 [J.Med.Chem, (1981), 24 753]. Ogilvy et al (Nucleosides and Nucleotides, (1983), 2(2), 147-154), reported that 1-[2-hydroxy-1-(hydroxymethyl) ethoxymethyl]cytosine, was tested against HSV-1 in a plaque reduction assay and found to have no activity at concentrations up to 100 ug. There is therefore no teaching or suggestion in the reference of any potential use of the compound in human medical therapy.

It has now surprisingly been found that 1-[2-hydroxy-1-(hydroxymethyl)methoxymethyl]cytosine, which may be represented by the formula (I)

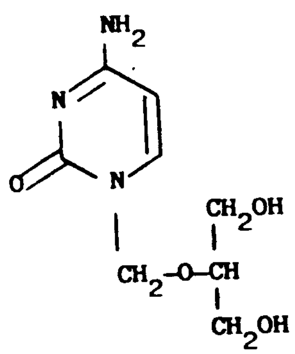

(I)

and its physiologically acceptable salts and esters thereof (including physiologically acceptable salts of such esters), are useful in the treatment of certain viral infections as described below. Physiologically acceptable salts of the compound of formula (I) which may be conveniently used in therapy include physiologically acceptable salts of organic acids such as lactic, acetic, malic or p-toluenesulphuric acid as well as physiologically acceptable salts of mineral acids such as hydrochloric acid.

The compound of formula (I) and the physiologically acceptable salts and esters (and salts of such esters) thereof are especialy useful in the treatment of diseases caused by various DNA viruses, and in particular cytomegalovirus (CMV), Epstein-Barr virus (EBV), varicella zoster virus (VZV) and Hepatitis B virus. CMV causes encephalitis, mental retardation, blindness and deafness in newborns, pneumonitis in bone marrow transplant recipients and renal transplant recipients, thrombocytopenia purpura, CMV mononucleosis and hepatitis; EBV causes infectious mononucleosis; VZV causes chicken pox, pneumonitis, encephalitis, intravascular coagulopathy, disseminated zoster, hepatitis, shingles and keratitis. Hepatitis B virus is a DNA virus which is one of the causative agents of human hepatitis. In addition to its use in human medical therapy the compound of formula (I) its salts, esters and salts of such esters can be administered to other animals for the treatment or prophylaxis of viral diseases, e.g. in other mammals.

The method herein includes inhibiting the replication of CMV, EBV or VZV or hepatitis B virus in host cells of a mammal which comprises applying an effective virus replication inhibiting amount of the compound of formula (I), or a physiologically acceptable salt or ester (and salts of such esters) thereof, to the infected cells.

According to a further feature of the present invention there is provided the compound of formula (I) and physiologically acceptable salts and esters (and salts of such esters) thereof for use in the therapy, e.g., treatment or prophylaxis, of a viral disease in an animal, e.g., a mammal such as man.

The compound of formula (I) or its physiologically acceptable salts, esters and salts of such esters may be prepared by any of the methods known in the art for the preparation of the same or similar compounds e.g. as described hereinafter.

The invention further provides the use of the compound of formula (I) or its physiologically acceptable salts, esters and salts of such esters in the manufacture of a medication for the treatment of DNA virus infections, particularly hepatitis B, VZV, EBV or CMV.

It has also surprisingly been found that oral administration of an ester, particularly the dipivalate ester, of the compound of formula (I) gives rise to substantially higher body fluid levels of the compound of formula (I) than does the oral administration of the compound of formula (I) itself, as demonstrated by urine levels in mice and rats.

The present invention further provides as novel compounds the physiologically acceptable esters of the compound of formula (I) and physiologically acceptable salts of such esters.

The novel compounds of this aspect of the invention may be represented for example by formula (II)

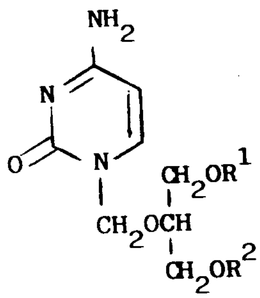

(II)

wherein $R^1$ and $R^2$ may be the same or different and each is selected from hydrogen and COR wherein R is selected from hydrogen, branched or straight chain or cyclic (including polycyclic) alkyl containing 1-12 carbons, phenyl or naphthyl ( $\alpha$- or $\beta$-) wherein said phenyl and naphthyl may be substituted with 1-3 substituents selected from $C_{1-4}$ alkyl, $C_{1-4}$ alkoxy and halo, providing that only one of $R^1$ and $R^2$ may be hydrogen, or $R^1$ and $R^2$ together with the oxygens to which they are attached form a carbonate moiety, and physiologically acceptable salts thereof.

Preferred compounds of formula (II) are those wherein $R^1$ and $R^2$ are COR and R is branched or straight chain alkyl containing 1-8 carbons or phenyl optionally substituted with 1-3 substituents selected from $C_{1-4}$ alkyl, $C_{1-4}$ alkoxy and halo, and physiologically acceptable salts thereof. Most preferred are those compounds of formula (II) wherein $R^1$ and $R^2$ are COR and R is branched or straight chain alkyl containing 1-4 carbons, e.g. tert-butyl.

As used herein halo includes fluoro, chloro and bromo.

Salts of the compound of formula (II) which may be conveniently used in therapy include physiologically acceptable salts of organic acids such as lactic, acetic, malic or p-toluenesulphonic acid as well as physiologically acceptable salts of mineral acids such as hydrochloric or sulphuric acid.

In a further aspect of the present invention is provided the use of compounds of formula (II) or physiologically acceptable salts thereof in the manufacture of a medication for the treatment of DNA virus infections, particularly VZV, EBV, CMV and hepatitis B virus infections.

The compounds of formula (II) may be prepared in conventional manner, e.g., using techniques as described in the Examples.

The present invention further provides a process for the preparation of compounds of formula (II) (as defined above) and physiologically acceptable salts thereof which comprises:

(a) reacting a compound of formula

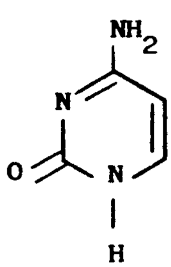

(III)

or a functional equivalent thereof, with a compound of formula

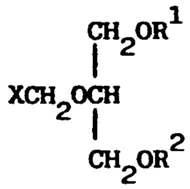

(IV)

(wherein the groups $R^1$ and $R^2$ are as defined above and X represents a leaving radical);

(b) reacting a compound of formula

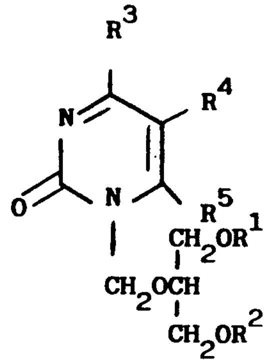

(V)

(wherein the groups $R^1$ and $R^2$ are as defined above; $R^3$ represents an amino group or a precursor group therefor; and $R^4$ and $R^5$ each represent a hydrogen atom or a precursor group therefor; at least one of $R^3$, and $R^4$ and $R^5$ representing a precursor group) with an agent serving to effect conversion of the said precursor group(s) to the desired group(s) $R^3$, $R^4$ and/or $R^5$;

(c) reacting a compound of formula

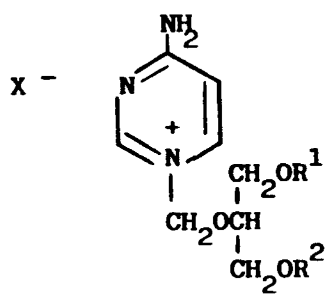

(VI)

(wherein the group $R^1$ and $R^2$ are as defined above and $X^-$ represents an anion) with an agent serving to effect conversion of the said compound of formula (VI) to the desired compound of formula (II);

(d) reacting a compound of formula

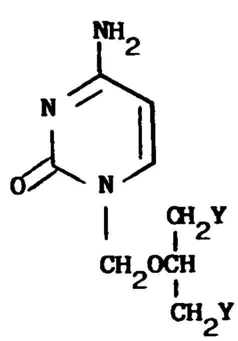

(VII)

(wherein each Y group represents a precursor for the -OCOR group as defined above) with an agent

serving to effect conversion of the said compound of formula (VII) to be desired compound of formula (II);

(e) effecting cyclisation of a precursor compound to form the cytosine ring system with consequent formation of a compound of formula (II); and optionally

(i) where the resulting product is a compound of formula (II), converting the said compound into a physiologically acceptable salt thereof; or

(ii) where the resulting product is a physiologically acceptable salt of a compound of formula (II), converting the said salt into the parent compound of formula (II).

With regard to process (a) above, the group X is advantageously a halo group, and the reaction is conveniently effected under basic conditions, e.g., in the presence of potassium carbonate or sodium hydroxide.

With regard to process (b), this may involve for example:

(i) conversion of a precursor group $R^3$ in the 6-position; and/or

(ii) conversion of precursor group(s) $R^4$ and/or $R^5$ in the 4- and 5-positions.

In the case of (i), the precursor group $R^3$ may be for example an azido group which can be converted into the desired amino group by catalytic hydrogenation preferably in the presence of palladium-charcoal, for example in an organic solvent medium such as methanol. The azido compound may be prepared for example from either (a) a corresponding alkylthio (e.g., methylthio) or alkylsulfonyl compound by reaction with an alkali metal (e.g., sodium) azide in an organic solvent medium such as aqueous ethanol, conveniently at an elevated temperature, e.g., up to about 80 ° C, or (b) a corresponding halo (e.g., chloro) compound by reaction with an alkali metal (e.g., lithium azide in an organic solvent medium such as dimethylformamide) conveniently at room temperature.

Further alternatives for the precursor group $R^3$ include for example:

(A) an oxo group which may be converted into the desired amino group by treatment with an ammonium salt such as ammonium sulphate, hexamethyldisilazane and formamide;

(B) an alkoxycarbonylamino group such as 2-(4-nitrophenyl)ethoxycarbonylamino which may be converted into the desired amino group by treatment with 1,5-diazabicyclo[4.3.0]non-5-ene or 1,5-diazabicyclo[5.4.0]undec-5-ene in an aprotic solvent such as pyridine; or

(C) a group of formula $-N=CHNMe_2$ which may be converted into the desired amino group by treatment with an alkanol such as ethanol or isopropanol at an elevated temperature.

With regard to process (c), this may be effected for example by treatment of the compound of formula (VI) with a xanthine oxidase enzyme. The compound of formula (VI) may be prepared by reacting a compound of formula

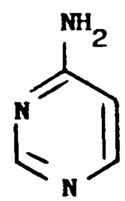

with a compound of formula (IV) under appropriate conditions, e.g., in dimethylformamide solution.

With regard to process (d), this may comprise for example, esterification (or transesterification) of a compound of formula (I), as hereinbefore defined, with an appropriate esterifying agent, e.g., an acid halide (preferably chloride) or anhydride, in a basic medium such as dimethylformamide/pyridine, or when transesterification is to be effected a lower alkyl ester in the presence of a base such as potassium carbonate.

When the desired ester of formula (II) is the carbonate,

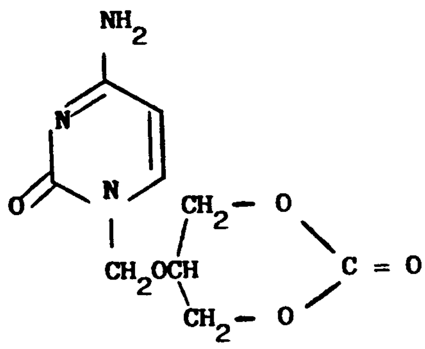

process (d) is conveniently used wherein reaction is effected between a compound of formula (I) and phosgene in a basic medium or a dialkyl carbonate in the presence of a base.

Alternatively Y in formula (VII) may each represent a halogen atom and the compound of formula (VII) is reacted with an excess of a compound formula MOCOR in which M represents an alkali metal such as potassium, conveniently in the presence of dimethylsulfoxide at an elevated temperature. As a further alternative Y may each represent an optionally substituted (e.g. nitro substituted) benzyloxy group which may be converted into the desired group by treatment with the appropriate acid anhydride of formula $(RCO)_2O$, conveniently in the presence of a Lewis acid.

With regard to process e) this may be effected for example by reaction of a compound of formula

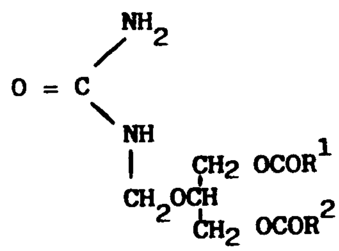

(wherein R is as defined above) with a compound of formula

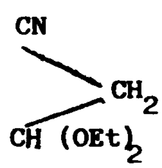

conveniently in the presence of an alkali metal alkoxide such as sodium ethoxide.

The compound of formula (I) and its physiologically acceptable salts and esters (including physysiologically acceptable salts of such esters) (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or veterinarian. In general, however, for each of these utilities and indications, a suitable, effective dose will be in the range 0.1 to 250 mg per kilogram body weight of recipient per day, preferably in the range 1 to 100 mg per kilogram body weight per day and most preferably in the range 5 to 20 mg per kilogram body weight per day; an optimum dose is about 15 mg per kilogram body weight per day (unless otherwise indicated all weights of active ingredient are calculated as the parent compound of formula (I); for salts thereof the figures would be increased proportionately.) The desired dose is preferably presented as two,

three, four or more sub-doses administered at appropriate intervals throughout the day. These sub-doses may be adminstered in unit dosage forms, for example, containing 10 to 1000 mg, preferably 20 to 500 mg and most preferably 100 to 400 mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Example of such dermal penetration enhancers include dimethylsulphoxide and related analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferrred esters. These may be used alone or in combination depending on the properties

required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

Burkitt's lymphoma and nasopharyngeal carcinoma amongst other diseases have been associated with the presence of EBV [see "Viral Infections of Humans", edited by Alfred S. Evans, Second Edition, 1982, Plenum Publishing Corporation, New York, chapter 24 and 25]. In this same text, "Viral Infections of Humans", see chapter 8 entitled "Cytomegalovirus" and chapter 10 entitled "Epstein-Barr Virus" for descriptions of diseases caused by these viruses.

In antiviral tests the compound of formula (I) was found to have surprisingly good activity against CMV, EBV and VZV.

The following Examples illustrate the present invention.

Example 1

1-[2-Hydroxy-1-(hydroxymethyl)ethoxymethyl]cytosine

A mixture of 5.1 g (0.013 M) of 2-bromomethoxy-1,3-bis-(0-benzoyl)glycerol, 2.2 g (0.013 M) of 2,4-diethoxypyrimidine in 26 ml of dry dichloromethane and 1.98 g (0.014 M) anhydrous potassium carbonate was stirred at room temperature overnight. The mixture was filtered and the inorganic salts washed with benzene. The combined filtrate and washings were evaporated in vacuo and the residual oil purified by column chromatography on silica gel. Elution with 20/1 dichloromethane/ether removed impurities and the desired 2-hydroxy-4-ethoxy-1-(1,3-dibenzoylpropoxymethyl)pyrimidine was eluted with 1/1 ether/dichloromethane giving 5.5 g as a clear oil, (93% yield).

The clear oil was heated in a bomb with 80 ml of freshly prepared, saturated methanolic ammonia at 95°C for 18 hours. The evaporated mixture was purified by column chromatography on silica gel. Elution with 5% methanol in dichloromethane yielded, on evaporation, 1.1 g of benzamide. Elution with 1/1 methanol/dichloromethane, gave on evaporation, 2.0 g of solid which on recrystallization from ethanol-acetonitrile yielded 1.75 g of 1-[2-hydroxy-1-(hydroxymethyl)ethoxymethyl]cytosine, mp 145-148°C, (67%

yield). The compound gave satisfactory elemental analysis and the $C^{13}$ NMR and ultraviolet spectra were consistent with the desired structure.

Example 2

1-[2-Hydroxy-1-(hydroxymethyl)ethoxymethyl]cytosine-Alternative Route

A mixture of 2.0 g (1.31 x $10^{-2}$ M) of $N^4$-acetylcytosine, 1.33 g of ammonium sulfate and 90 ml of hexamethyldisilazane was refluxed under nitrogen with stirring for 18 hours. The excess solvent was removed by flash evaporation and the residual oil dissolved in 50 ml of dry benzene. To this solution was added 5.15 g (1.31x$10^{-2}$M) of 2-bromomethoxy-1,3-bis(0-benzoyl)glycerol and the resulting orange solution refluxed on a steam bath for 18 hours. The reaction solution was evaporated in vacuo and the residual oil treated with hot methanol. The methanol extracts were absorbed on silica gel and added to a column of silica gel for flash chromatography. After elution with 3-10% methanol in dichloromethane to remove impurities, elution with 20% methanol in dichloromethane yielded, on evaporation, 1.4 g (25%) of 1-[2-benzoyloxy-1-(benzoyl-oxymethyl)ethoxymethyl]cytosine. The solid was dissolved in methanol and an equivalent volume of 40% aqueous methylamine added. After stirring at room temperature for 1.5 hours, the solution was heated on a steam bath for 15 minutes and flash evaporated. The residue was dissolved in water and extracted with dichloromethane 3 times. The aqueous phase was evaporated in vacuo after treatment with Rexyn $OH^-$ resin. The residue was dissolved in ethanol, chilled and ethereal-HCl added to give a pH of 1.0. The resultant precipitate was filtered, washed with ether and dried to give 75 mg of 1-[2-hydroxy-1-(hydroxymethyl)ethoxymethyl]cytosine as a 1/2 HCl salt. Analysis (CNH) and spectra (NMR) were consistent with the structure. M.p. = 164-166 °C.

Example 3

2-[(4-Amino-1,2-dihydro-2-oxo-1-pyrimidinyl)methoxy]-1,3-propanediyl Dibenzoate

A mixture of cytosine (0.666 g) and hexamethyldisilazane (4.5 ml) was refluxed one hour under nitrogen with magnetic stirring. The resulting clear solution was evaporated in vacuo, and the white fluffy solid combined with 2-(bromomethoxy)-1,3-propanediyl dibenzoate (1.59 g). The mixture was heated at 140-160 °C at 19995 $Nm^{-2}$ (150 mm kg) for 30 minutes. After about 10 minutes heating the mixture fused into a light orange viscous oil. After cooling to room temperature, the oil was dissolved in 1:1 (v/v) dichloromethane:methanol and stirred at room temperature for 15 minutes. The mixture was filtered, the insoluble precipitate discarded and the mother liquor preabsorbed on silica gel and evaporated in vacuo. The residual powder was added to a column prepared for flash chromatography and the column was eluted with, first, two litres of 5% methanol in dichloromethane. The desired product was then eluted with one litre more of the eluant to yield, on evaporation, 0.8 g of the title compound. Recrystallization from hot benzene gave 0.60 g (35%) of analytically pure 2-[(4-amino-1,2-dihydro-2-oxo-1-pyrimidinyl)methoxy]-1,3-propanediyl dibenzoate, m.p. = 152-153 °C having a satisfactory $^1$H-NMR spectrum.

Example 4

(a) 2-(Methoxymethoxy)-1,3-propanediyl Diacetate

A mixture of 1,3-dichloro-2-(methoxymethoxy)propane (181.1 g), potassium acetate (308 g), and 18-crown-6 (19.0 g) in dry dimethylsulfoxide (500 ml) was stirred at 77 °C for 18 hours. The resulting two phase system was cooled to room temperature, which caused the lower layer to partially solidify. The mixture was filtered and the filtrate evaporated in vacuo. The residue was partitioned between ether and water, and the ethereal layer was extracted twice more with water. After drying over sodium sulfate, the organic extracts were filtered and evaporated to give yellow liquid which was distilled. The distillate was 2-(methoxymethoxy)-1,3-propanediyl diacetate b.p. 114-119 °C/1.5 mm, 113.1 g (49.1%). $^1$H $^{13}$C NMR spectra as well as mass-spectroscopy confirmed this structure.

(b) 2-(Acetoxymethoxy)-1,3-propanediyl Diacetate

To a cooled (0 °C) solution of 2-(methoxymethoxy)-1,3-propanediyl diacetate (25.0 g) in acetic anhydride (33.1 ml) was added dropwise boron trifluoride etherate (5.2 ml) over a 10 minute period. The

resultant yellow solution was stirred 5 hours at 0°C and then poured into a suspension of sodium bicarbonate (29.5 g) in $H_2O$ (200 ml) at 0°C. The mixture was stirred until the foaming subsided, then extracted four times with 100 ml portions of ether. The combined ethereal extracts were washed with saturated sodium bicarbonate solution, dried over sodium sulfate, filtered and evaporated to give a yellow oil 21.3 g (75%). The $^1H$ NMR spectrum was consistent with the desired product, 2-(acetoxymethoxy)-1,3-propanediyl diacetate.

(c) 2-(Bromomethoxy)-1,3-propanediyl Diacetate

A solution of 2-(acetoxymethoxy)-1,3-propanediyl diacetate (21.3 g), trimethylsilybromide (46 ml) and dry dichloromethane (200 ml) was refluxed for 18 hours. The solution was flash evaporated to yield 21.5 g (93%) of a pale yellow liquid whose $^1H$ NMR spectrum was consistent with the desired structure, 2-bromomethoxy-1,3-propanediyl diacetate.

(d) 2-[(4-Amino-1,2-dihydro-2-oxo-1-pyrimidinyl)methoxy]-1,3-propanediyl Diacetate

A mixture of cytosine (1.03 g) and hexamethyldisilazane (6.7 ml) was refluxed for 1.75 hours. The solution was evaporated in vacuo and the residue combined with 2-(bromomethoxy)-1,3-propanediyl diacetate (1.62 g). The mixture was heated at 180°C at water aspirator pressure for one hour. The orange brown oil was cooled and warmed with 20 ml of methanol. A small amount (40 mg) of insoluble solid was filtered off and the filtrate was evaporated, redissolved in dichloromethane and filtered again to remove the unreacted cytosine. The final filtrate was evaporated and purified by flash column chromatography. The desired product was eluted with 2.5 liters of 10% methanol in dichloromethane, with the middle fractions containing the title compound. After evaporation of the pooled fractions, the light brown-yellow solid was dissolved in 150 ml of methanol-dichloromethane and treated with 10 mg of charcoal (Norit Registered Trademark). The solution was filtered through a pad of celite and concentrated to yield, on cooling, 148 mg (8.2%) of a pale yellow solid, 2-[(4-amino-1,2-dihydro-2-oxo-1-pyrimidinyl)methoxy]-1,3-propanediyl diacetate, m.p. = 151-154°C. The $^1H$ NMR spectrum and elemental analysis supported this structure.

Example 5

(a) 2-(Methoxymethoxy)-1,3-propanediyl Dipivalate

A mixture of 1,3-dichloro-2-(methoxymethoxy)propane (17.3 g), potassium pivalate (56.1 g) and dry dimethylsulfoxide (400 ml) was stirred with heating at 70°C (internal temperature) for 18 hours. After standing at room temperature for four days, the mixture was filtered and the filtrate evaporated in vacuo. The residue was partitioned between water and ether and the aqueous phase extracted twice more with ether. The combined ethereal extracts were dried over sodium sulfate, filtered and evaporated to yield 29.4 g (97%) of a pale yellow liquid, 2-(methoxymethoxy)-1,3-propanediyl dipivalate. The $^1H$ and $^{13}C$ NMR spectra were consistent with the desired structure.

(b) 2-(Acetoxymethoxy)-1,3-propanediyl Dipivalate

A solution of 2-(methoxymethoxy)-1,3-propanediyl dipivalate (29.0 g) and acetic anhydride (11 ml) was chilled in an ice bath to 0°C, and boron trifluoride ethereate (2.86 ml) was added dropwise to the solution with stirring at 0°C over a 3 hour period.

A solution was poured into 160 ml ice and water containing 128 g of sodium bicarbonate. The mixture was stirred until the ice melted and then extracted three times with equal volumes of ether. The combined ether extracts were washed with a saturated solution of sodium bicarbonate, and the ethereal layer dried over sodium sulfate, filtered and evaporated at 60°C. The resulting colorless oil, 2-(acetoxymethoxy)-1,3-propanediyl dipivalate (26.4 g, 84%), was analysed by $^1H$ and $^{13}C$ NMR and showed 99% purity.

(c) 2-(Bromomethoxy)-1,3-propanediyl Dipivalate

A solution of 2-(acetoxymethoxy)-1,3-propanediyl dipivalate (26.4 g), dry dichloromethane (124 ml) and trimethylsilyl bromide (30.1 ml) was refluxed for two hours, then evaporated in vacuo. The residual yellow oil, 2-(bromomethoxy)-1,3-propanediyl dipivalate, 27.3 g, was sufficiently pure by $^1H$ NMR to use without purification; the yield was 98.0%.

(d) 2-[(4-Amino-1,2-dihydro-2-oxo-1-pyrimidinyl)methoxy]-1,3-propanediyl Dipivalate

A mixture of cytosine (6.5 g) and hexamethyldisilazane (43 ml) was refluxed with magnetic stirring for one hour. The solution was evaporated in vacuo, and the resulting white solid was combined with 2-(bromomethoxy)-1,3-propanediyl dipivalate (13.75 g) and the mixture heated at 140-150° C at 20 mm (water aspirator pressure) for 30 minutes. The amber oil was cooled and dissolved in dichloromethane (400 ml) and 80% aqueous methanol (1:1 v/v). The solution was stirred at room temperature for 15 minutes and then evaporated in vacuo. The residue was dissolved in chloroform, and the insoluble material (2.0 g) filtered off. The chloroform filtrate was washed twice with water, and the organic extracts were dried over sodium sulfate. The evaporated extracts yielded a mixture of products which was purified by flash column chromatography on silica gel. The column was eluted with two liters of 1:1 (v/v) ethyl acetate:dichloromethane and the desired product was obtained from evaporation of the 10% methanol:dichloromethane eluate (two liters). Recrystallization of the residue from benzene:hexane gave 6 g (40%) of 2-[(4-amino-1,2-dihydro-2-oxo-1-pyrimidinyl)methoxy]-1,3-propanediyl dipivalate, m.p. = 153-154° C with satisfactory elemental analyses and $^1$H and $^{13}$C NMR spectra.
In the following Examples 6-9 inclusive, the compound of formula (II) is taken as 2-[(4-amino-1,2-dihydro-2-oxo-1-pyrimidinyl)methoxy]-1,3-propanediyl dipivalate, i.e. $R^1 = R^2 = COR$ and $R = C(CH_3)_3$.

Example 6

Toxicity

The compound of formula (II) was administered to 28 male CD-1 mice by gavage. The dose was 44.5 mg per kg of body weight and was in suspension in 5% Tween 80 at a concentration of 5 mg/ml. No signs of toxicity were observed in any of the mice.

Example 7

Tablet

| | |
|---|---|
| Compound of formula (II) | 100 mg |
| Lactose | 200 mg |
| Starch | 50 mg |
| Polyvinylpyrrolidone | 5 mg |
| Magnesium stearate | 4 mg |
| | ------- |
| | 359 mg |

Tablets were prepared from the foregoing ingredients by wet granulation followed by compression.

Example 8

| Injectable Solution | |
|---|---|
| Hydrochloride salt of compound of Formula (II) | 0.775 g |
| Sterile, pyrogen-free, pH 7 phosphate buffer to | 25 ml |

Example 9

| Ophthalmic Solution | |
|---|---|
| Hydrochloride salt of compound of Formula (II) | 1.0 g |
| Sodium chloride, analytical grade | 0.9 g |
| Thiomersal | 0.001 g |
| Purified water to | 100 ml |
| pH adjusted to | 5.5-7.5 |

Example 10

## In vitro CMV activity of 1-[2-hydroxy-1-(hydroxymethyl)ethoxymethyl]cytosine (Plaque Reduction Assay)

Confluent monolayers of human diploid fibroblast cells (i.e., human foreskin fibroblast cells) were infected with 200-300 $\mu$L of a suspension containing 50-100 human CMV infectious virions per culture well and allowed to incubate for 90 minutes at 37 degrees C in a $CO_2$ incubator. Unadsorbed virus was then removed by aspiration and appropriate concentrations of 1-[2-hydroxy-1-(hydroxymethyl)ethoxymethyl] cytosine were added in Eagle's Minimal Essential Media containing 2% fetal calf serum in an agarose overlay (0.6%). The assay plates were incubated at 37 degrees C in a $CO_2$ incubator for 8 days to allow plaque development. The monolayers were then fixed with 10% formalin in phosphate buffered saline and stained wth 0.8% crystal violet. The plaques were counted at 3x magnification using a Bausch and Lomb dissecting microscope, and the data were analysed by SAS Probit (SAS Institute, Inc., Cary, N.C., U.S.A.) to give the following results:

| Virus Strain | Cell Line | $ED_{50}$ ($\mu$M) |
|---|---|---|
| ATCC VR-538 (strain AD169) | Human Foreskin Fibroblasts | 3.7 ± 0.7 |
| Kerr | Human Foreskin Fibroblasts | 1.9 ± 0.2 |

Example 11

## In vitro EBV activity of 1-[2-hydroxy-1-(hydroxymethyl)ethoxymethyl]cytosine (cRNA-DNA Hybridization Technique)

1-[2-Hydroxy-1-(hydroxymethyl)ethoxymethyl]cytosine was assayed for activity against Epstein-Barr Virus (EBV) using an adaptaion of the procedure described in Nature New Biology, 233, 103-106 (1971). This technique, employing an EBV-specific cRNA-DNA hybridization on filter membranes, detects the amount of EBV genome equivalents produced in the virus-producing cell lines. In this assay the EBV-producing cell line P3HR-1 (ATCC HTB 62) was exposed to various concentrations of 1-[2-hydroxy-1-(hydroxymethyl)-ethoxymethyl]cytosine for 14 days and EBV genome equivalents per cell were then determined to give an $ED_{50}$ of approximately 0.05 $\mu$M.

**Claims**

**1.** A pyrimidine derivative selected from 1-[2-hydroxy-1-(hydroxymethyl)ethoxymethyl]cytosine and physiologically acceptable salts or esters thereof (and physiologically acceptable salts of such esters) for use in therapy.

2. A pyrimidine derivative as claimed in claim 1 for use in the treatment or prophylaxis of a viral disease in an animal.

3. A pyrimidine derivative as claimed in claim 2 for use in the treatment or prophylaxis of hepatitis B, varicella zoster virus, Epstein-Barr virus or cytomegalovirus.

4. A pyrimidine derivative as claimed in any of claims 1 to 3 having the formula (II)

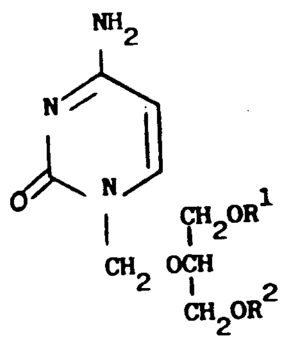

wherein $R^1$ and $R^2$ may be the same or different and each is selected from hydrogen and COR wherein R is selected from hydrogen, branched or straight chain or cyclic (including polycyclic) alkyl containing 1-12 carbons, phenyl or naphthyl ( $\alpha$- or $\beta$-) wherein said phenyl or naphthyl may be substituted with 1-3 substituents selected from $C_{1-4}$ alkyl, $C_{1-4}$ alkoxy and halo, provided only one of $R^1$ and $R^2$ may be hydrogen, or $R^1$ and $R^2$ together with the oxygens to which they are attached form a carbonate moiety, or a physiologically acceptable salt thereof.

5. A pyrimidine derivative as claimed in claim 4 wherein $R^1$ and $R^2$ are COR and R is branched or straight chain alkyl containing 1-8 carbons or phenyl optionally substituted with 1-3 substituents selected from $C_{1-4}$ alkyl, $C_{1-4}$ alkoxy and halo, or a physiologically acceptable salt thereof.

6. A pyrimidine derivative as claimed in claim 5 wherein R is branched or straight chain $C_{1-4}$ alkyl.

7. Use of a pyrimidine derivative selected from 1-[2-hydroxy-1-(hydroxymethyl)ethoxymethyl]cytosine or a physiologically acceptable salt or ester thereof (including a physiologically acceptable salt of such an ester) for the preparation of a pharmaceutical formulation for the treatment or prophylaxis of a DNA virus infection.

8. Use of a pyrimidine derivative as claimed in claim 7 for the preparation of a pharmaceutical formulation for the treatment or prophylaxis of a hepatitis B, varicella zoster virus, Epstein-Barr virus or cytomegalovirus infection.

9. Pharmaceutical formulations comprising at least one pyrimidine derivative selected from 1-[2-hydroxy-1-(hydroxymethyl)ethoxymethyl]cytosine or a physiologically acceptable salt or ester thereof (or a physiologically acceptable salt of such an ester), together with one or more pharmaceutical carriers.

10. Pharmaceutical formulations as claimed in claim 9 in the form of tablets, capsules or a sterile preparation.

11. A compound of formula (II)

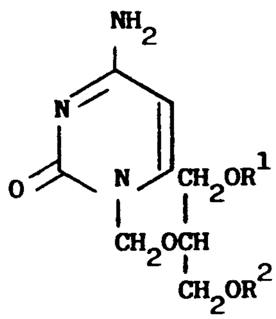

(II)

wherein $R^1$ and $R^2$ may be the same or different and each is selected from hydrogen and COR wherein R is selected from hydrogen, branched or straight chain or cyclic (including polycyclic) alkyl containing 1-12 carbons, phenyl or naphthyl ( $\alpha$- or $\beta$-) wherein said phenyl or naphthyl may be substituted with 1-3 substituents selected from $C_{1-4}$ alkyl, $C_{1-4}$ alkoxy and halo, provided that only one of $R^1$ and $R^2$ may be hydrogen, or $R^1$ and $R^2$ together with the oxygens to which they are attached form a carbonate moiety, or a physiologically acceptable salt thereof.

**12.** A compound according to claim 11 wherein $R^1$ and $R^2$ are COR and R is branched or straight chain alkyl containing 1-8 carbons or phenyl optionally substituted with 1-3 substituents selected from $C_{1-4}$ alkyl, $C_{1-4}$ alkoxy and halo, or a physiologically acceptable salt thereof.

**13.** A compound according to claim 12 wherein $R^1$ and $R^2$ are COR and R is branched or straight chain $C_{1-4}$ alkyl.

**14.** A process for the preparation of compounds according to any of claims 11,12 and 13 and physiologically acceptable salts thereof which comprises:

(a) reacting a compound of formula (III)

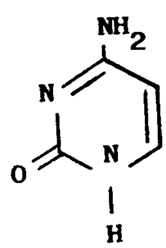

(III)

or a functional equivalent thereof, with a compound of formula (IV)

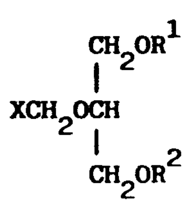

(IV)

(wherein the groups $R^1$ and $R^2$ are as defined and X represents a leaving radical); or

(b) reacting a compound of formula (V)

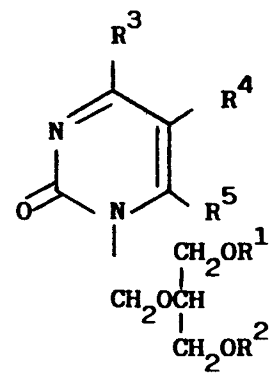

(V)

(wherein the groups $R^1$ and $R^2$ are as defined; $R^3$ represents an amino group or a precursor group therefor; and $R^4$ and $R^5$ each represent a hydrogen atom or a precursor group therefor; at least one of $R^3$, and $R^4$ and $R^5$ representing a precursor group) with an agent serving to effect conversion of the said precursor group(s) to the desired group(s) $R^3$, $R^4$ and/or $R^5$; or

(c) reacting a compound of formula (VI)

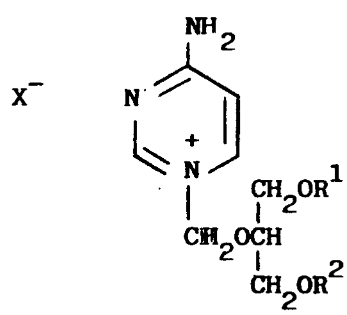

(VI)

(wherein the group $R^1$ and $R^2$ are as defined and $X^-$ represents an anion) with an agent serving to effect conversion of the said compound of formula (VI) to the desired compound of formula (II); or

(d) reacting a compound of formula (VII)

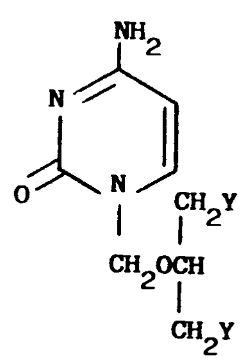

(VII)

(wherein each Y group represents a precursor for the -OCOR group as defined) with an agent serving to effect conversion of the said compound of formula (VII) to the desired compound of formula (II); or

(e) effecting cyclisation of a precursor compound to form the cytosine ring system with consequent formation of a compound of formula (II); and optionally

(i) where the resulting product is a compound of formula (II), converting the said compound into a physiologically acceptable salt thereof;

(ii) where the resulting product, is a physiologically acceptable salt of a compound of formula (II), converting the said salt into the parent compound of formula (II).

## Revendications

1. Dérivé de pyrimidine choisi parmi la 1-[2-hydroxy-1-(hydroxyméthyl)-éthoxyméthyl]-cytosine et ses sels ou esters physiologiquement acceptables (et les sels physiologiquement acceptables de ces esters) à utiliser en thérapeutique.

2. Dérivé de pyrimidine suivant la revendication 1, à utiliser dans le traitement ou la prophylaxie d'une affection virale chez un animal.

3. Dérivé de pyrimidine suivant la revendication 2, à utiliser dans le traitement ou la prophylaxie de l'hépatite B, du virus de la varicelle zoster, du virus d'Epstein-Barr ou du cytomégalovirus.

4. Dérivé de pyrimidine suivant l'une quelconque des revendications 1 à 3, de formule (II) :

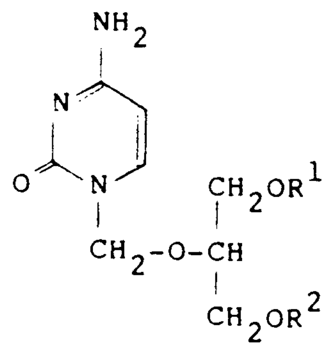

où $R^1$ et $R^2$ peuvent être identiques ou différents et sont choisis chacun entre hydrogène et COR, où R est choisi entre hydrogène, alcoyle en chaîne droite ou ramifiée ou cyclique (y compris polycyclique) comptant 1 à 12 atomes de carbone, phényle et naphtyle ($\alpha$ ou $\beta$), où le phényle et le naphtyle peuvent être substitués par un à trois substituants choisis parmi $C_{1-4}$-alcoyle, $C_{1-4}$-alcoxy et halo, avec la restriction qu'un seul d'entre $R^1$ et $R^2$ peut être hydrogène, ou bien $R^1$ et $R^2$ conjointement avec les oxygènes auxquels ils sont unis forment un radical carbonate,
ou un sel physiologiquement acceptable de celui-ci.

5. Dérivé de pyrimidine suivant la revendication 4, dans lequel $R^1$ et $R^2$ sont COR et R est alcoyle en chaîne droite ou ramifiée de 1 à 8 atomes de carbone ou phényle facultativement substitué par 1 à 3 substituants choisis parmi $C_{1-4}$-alcoyle, $C_{1-4}$-alcoxy et halo,
ou un sel physiologiquement acceptable de celui-ci.

6. Dérivé de pyrimidine suivant la revendication 5, dans lequel R est $C_{1-4}$-alcoyle.

7. Utilisation d'un dérivé de pyrimidine choisi parmi la 1-[2-hydroxy-1-(hydroxyméthyl)-éthoxyméthyl]-cytosine et un sel ou ester physiologiquement acceptable de celle-ci (y compris un sel physiologiquement acceptable d'un tel ester) pour la préparation d'une composition pharmaceutique destinée au traitement ou à la prophylaxie d'une infection par un virus à ADN.

8. Utilisation d'un dérivé de pyrimidine suivant la revendication 7 pour la préparation d'une composition pharmaceutique destinée au traitement ou à la prophylaxie de l'hépatite B ou d'une infection par le virus de la varicelle zoster, le virus d'Epstein-Barr ou le cytomégalovirus.

9. Compositions pharmaceutiques comprenant au moins un dérivé de pyrimidine choisi parmi la 1-[2-hydroxy-1-(hydroxyméthyl)-éthoxyméthyl]-cytosine et un sel ou ester physiologiquement acceptable de celle-ci (ou un sel physiologiquement acceptable d'un tel ester) conjointement avec un ou plusieurs excipients pharmaceutiques.

10. Compositions pharmaceutiques suivant la revendication 9, sous la forme de comprimés, de capsules ou de préparations stériles.

**11.** Composé de formule (II) :

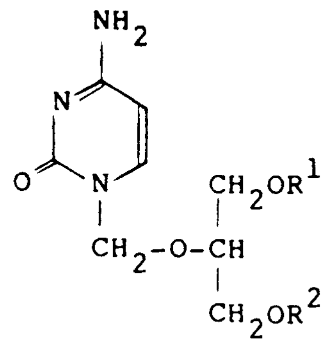

où $R^1$ et $R^2$ peuvent être identiques ou différents et sont choisis chacun entre hydrogène et COR, où R est choisi entre hydrogène, alcoyle en chaîne droite ou ramifiée ou cyclique (y compris polycyclique) comptant 1 à 12 atomes de carbone, phényle et naphtyle ($\alpha$ ou $\beta$), où le phényle et le naphtyle peuvent être substitués par un à trois substituants choisis parmi $C_{1-4}$-alcoyle, $C_{1-4}$-alcoxy et halo, avec la restriction qu'un seul d'entre $R^1$ et $R^2$ peut être hydrogène, ou bien $R^1$ et $R^2$ conjointement avec les oxygènes auxquels ils sont unis forment un radical carbonate, ou un sel physiologiquement acceptable de celui-ci.

**12.** Composé suivant la revendication 11, dans lequel $R^1$ et $R^2$ sont COR et R est alcoyle en chaîne droite ou ramifiée de 1 à 8 atomes de carbone ou phényle facultativement substitué par 1 à 3 substituants choisis parmi $C_{1-4}$-alcoyle, $C_{1-4}$-alcoxy et halo, ou un sel physiologiquement acceptable de celui-ci.

**13.** Composé suivant la revendication 12, dans lequel $R^1$ et $R^2$ sont COR et R est $C_{1-4}$-alcoyle en chaîne droite ou ramifiée.

**14.** Procédé de préparation de composés suivant l'une quelconque des revendications 11, 12 et 13, et de leurs sels physiologiquement acceptables, qui comprend :

(a) la réaction d'un composé de formule (III) :

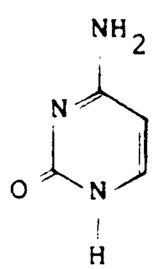

(III)

ou d'un équivalent fonctionnel de celui-ci, avec un composé de formule (IV) :

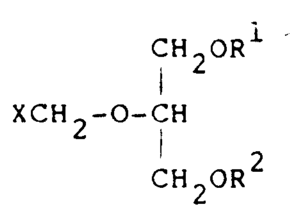

(IV)

(où $R^1$ et $R^2$ sont tels que définis et X représente un radical partant);

(b) la réaction d'un composé de formule (V) :

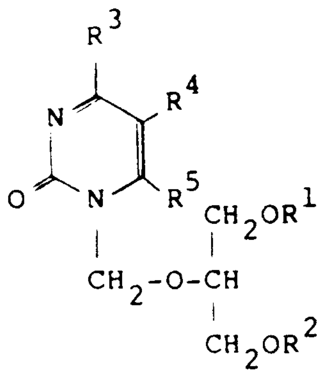

(V)

(où $R^1$ et $R^2$ sont tels que définis ci-dessus; $R^3$ représente un radical amino ou un radical précurseur de celui-ci et $R^4$ et $R^5$ représentent chacun un atome d'hydrogène ou un radical précurseur de celui-ci; au moins l'un d'entre $R^3$, et $R^4$ et $R^5$ représentant un radical précurseur) avec un agent servant à opérer la conversion du ou des radicaux précurseurs en le ou les radicaux souhaités $R^3$, $R^4$ et/ou $R^5$;

(c) la réaction d'un composé de formule (VI) :

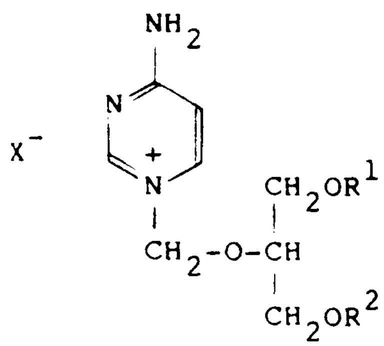

(VI)

(où $R^1$ et $R^2$ sont tels que définis ci-dessus et $X^-$ représente un anion) avec un agent servant à opérer la conversion du composé de formule (VI) en le composé souhaité de formule (II);

(d) la réaction d'un composé de formule (VII) :

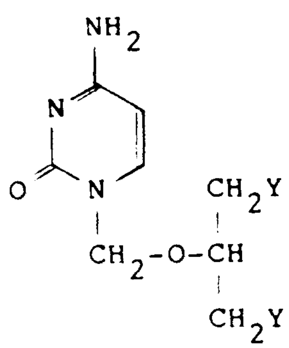

(VII)

(où chaque Y représente un précurseur du radical -OCOR tel que défini) avec un agent servant à opérer la conversion du composé de formule (VII) en le composé souhaité de formule (II);

(e) l'exécution de la cyclisation d'un composé précurseur pour former le système cyclique de la cytosine avec formation résultante d'un composé de formule (II); et facultativement

(i) lorsque le produit résultant est un composé de formule (II), la conversion de ce composé en un sel physiologiquement acceptable de celui-ci; ou

(ii) lorsque le produit résultant est un sel physiologiquement acceptable d'un composé de formule

(II), la conversion de ce sel en le composé de formule (II) d'origine.

**Patentansprüche**

1. Pyrimidinderivat ausgewählt aus 1-[2-Hydroxy-1-(hydroxymethyl)-äthoxymethyl]-cytosin und physiologisch annehmbaren Salzen oder Estern hievon (und physiologisch annehmbaren Salzen derartiger Ester) zur therapeutischen Verwendung.

2. Pyrimidinderivat nach Anspruch 1 zur Verwendung bei der Behandlung oder Prophylaxe einer Viruserkrankung in einem Lebewesen.

3. Pyrimidinderivat nach Anspruch 2 zur Verwendung bei der Behandlung oder Prophylaxe von Hepatitis B, Varicella zoster-Virus, Epstein-Barr-Virus oder Zytomegalie-Virus.

4. Pyrimidinderivat nach einem der Ansprüche 1 bis 3 der Formel (II)

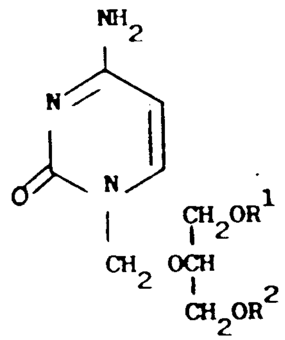

(II) ,

worin $R^1$ und $R^2$ gleich oder verschieden sein können und jeweils ausgewählt sind aus Wasserstoff und COR, wobei R ausgewählt ist aus Wasserstoff, verzweigt- oder geradkettigem oder cyclischem (einschließlich polycyclischem) Alkyl mit 1 bis 12 Kohlenstoffatomen, Phenyl oder Naphthyl, wobei das Phenyl oder Naphthyl mit 1 bis 3 Substituenten ausgewählt aus $C_1$-$C_4$-Alkyl, $C_1$-$C_4$-Alkoxy und Halogen substituiert sein kann, mit der Maßgabe, daß nur eines von $R^1$ und $R^2$ Wasserstoff sein kann, oder $R^1$ und $R^2$ zusammen mit den Sauerstoffatomen, an die sie gebunden sind, eine Carbonatgruppe bilden, oder ein physiologisch annehmbares Salz hievon.

5. Pyrimidinderivat nach Anspruch 4, worin $R^1$ und $R^2$ COR sind und R gerad- oder verzweigtkettiges Alkyl mit 1 bis 8 Kohlenstoffatomen oder Phenyl, gegebenenfalls substituiert mit 1 bis 3 Substituenten ausgewählt aus $C_1$-$C_4$-Alkyl, $C_1$-$C_4$-Alkoxy und Halogen, bedeutet oder ein physiologisch annehmbares Salz hievon.

6. Pyrimidinderivat nach Anspruch 5, worin R gerad- oder verzweigtkettiges $C_1$-$C_4$-Alkyl darstellt.

7. Verwendung eines Pyrimidinderivats ausgewählt aus 1-[2-Hydroxy-1-(hydroxymethyl)-äthoxymethyl]-cytosin oder einem physiologisch annehmbaren Salz oder Ester hievon (einschließlich einem physiologisch annehmbaren Salz eines derartigen Esters) zur Herstellung einer pharmazeutischen Formulierung zur Behandlung oder Prophylaxe einer DNA-Virusinfektion.

8. Verwendung eines Pyrimidinderivats nach Anspruch 7 zum Herstellen einer pharmazeutischen Formulierung zur Behandlung oder Prophylaxe einer Hepatitis B-, Varicella zoster-Virus-, Epstein-Barr-Virus- oder Zytomegalie-Virusinfektion.

9. Pharmazeutische Formulierungen umfassend wenigstens ein Pyrimidinderivat ausgewählt aus 1-[2-Hydroxy-1-(hydroxymethyl)-äthoxymethyl]-cytosin oder einem physiologisch annehmbaren Salz oder Ester hievon (oder einem physiologisch annehmbaren Salz eines derartigen Esters) zusammen mit einem oder mehreren pharmazeutischen Trägern.

**10.** Pharmazeutische Formulierung nach Anspruch 9 in Form von Tabletten, Kapseln oder eines sterilen Präparats.

**11.** Verbindung der Formel (II)

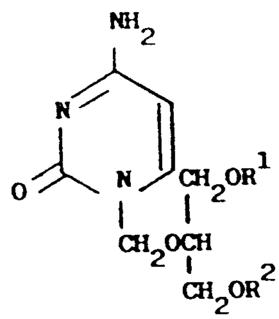

(II) ,

worin $R^1$ und $R^2$ gleich oder verschieden sein können und jeweils ausgewählt sind aus Wasserstoff und COR, wobei R ausgewählt ist aus Wasserstoff, verzweigt- oder geradkettigem oder cyclischem (einschließlich polycyclischem) Alkyl mit 1 bis 12 Kohlenstoffatomen, Phenyl oder Naphthyl ($\alpha$- oder $\beta$-), wobei das Phenyl oder Naphthyl mit 1 bis 3 Substituenten ausgewählt aus $C_1$-$C_4$-Alkyl, $C_1$-$C_4$-Alkoxy und Halogen substituiert sein kann, mit der Maßgabe, daß nur eines von $R^1$ und $R^2$ Wasserstoff sein kann, oder $R^1$ und $R^2$ zusammen mit den Sauerstoffatomen, an die sie gebunden sind, eine Carbonatgruppe bilden, oder ein physiologisch annehmbares Salz hievon.

**12.** Verbindung nach Anspruch 11, worin $R^1$ und $R^2$ COR sind und R verzweigt- oder geradkettiges Alkyl mit 1 bis 8 Kohlenstoffatomen oder Phenyl gegebenenfalls substituiert mit 1 bis 3 Substituenten ausgewählt aus $C_1$-$C_4$-Alkyl, $C_1$-$C_4$-Alkoxy und Halogen bedeutet, oder ein physiologisch annehmbares Salz hievon.

**13.** Verbindung nach Anspruch 12, worin $R^1$ und $R^2$ COR bedeuten und R verzweigt- oder geradkettiges $C_1$-$C_4$-Alkyl darstellt.

**14.** Verfahren zur Herstellung von Verbindungen nach einem der Ansprüche 11, 12 und 13 und physiologisch annehmbaren Salzen hievon, umfassend:

(a) das Umsetzen einer Verbindung der Formel (III)

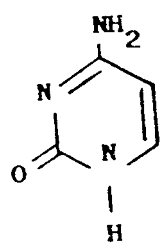

(III)

oder eines funktionellen Äquivalents hievon mit einer Verbindung der Formel (IV)

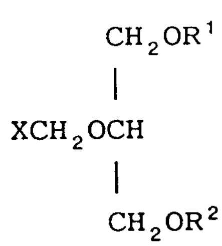

(IV)

(worin die Gruppen $R^1$ und $R^2$ wie definiert sind und X eine Abgangsgruppe darstellt), oder
(b) das Umsetzen einer Verbindung der Formel (V)

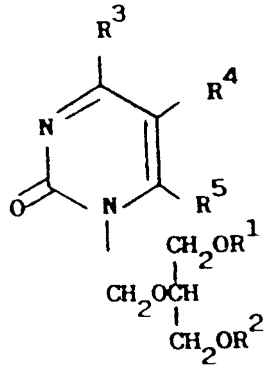

(V)

(worin die Gruppen $R^1$ und $R^2$ wie definiert sind, $R^3$ eine Aminogruppe oder eine Vorläufergruppe hiefür darstellt und $R^4$ und $R^5$ jeweils ein Wasserstoffatom oder eine Vorläufergruppe hiefür bedeuten, wobei wenigstens eines von $R^3$ und $R^4$ und $R^5$ eine Vorläufergruppe ist) mit einem Mittel, das zum Bewirken der Überführung der Vorläufergruppe(n) in die gewünschte(n) Gruppe(n) $R^3$, $R^4$ und/oder $R^5$ dient, oder
(c) das Umsetzen einer Verbindung der Formel (VI)

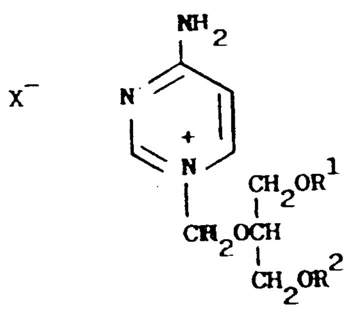

(VI)

(worin die Gruppen $R^1$ und $R^2$ wie definiert sind und $X^-$ ein Anion darstellt) mit einem Mittel, das zum Bewirken der Überführung der Verbindung der Formel (VI) in die gewünschte Verbindung der Formel (II) dient, oder
(d) das Umsetzen einer Verbindung der Formel (VII)

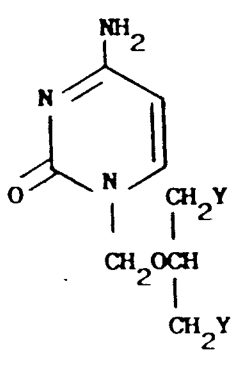

(VII)

(worin jede Gruppe Y einen Vorläufer für die -OCOR-Gruppe, wie oben definiert, darstellt) mit einem Mittel, das zum Bewirken der Überführung der Verbindung der Formel (VII) in die gewünschte Verbindung der Formel (II) dient, oder
(e) das Bewirken der Cyclisierung einer Vorläuferverbindung zur Bildung des Cytosinringsystems mit entsprechender Bildung einer Verbindung der Formel (II); und gegebenenfalls

(i) wenn das erhaltene Produkt eine Verbindung der Formel (II) ist, das Überführen der Verbindung in ein physiologisch annehmbares Salz hievon;
(ii) wenn das erhaltene Produkt ein physiologisch annehmbares Salz einer Verbindung der Formel (II) ist, das Überführen des Salzes in die Stammverbindung der Formel (II).